(19) Europäisches Patentamt
European Patent Office
Office européen des brevets

(11) Publication number: 0 217 635
A2

(12) # EUROPEAN PATENT APPLICATION

(21) Application number: 86307359.9

(22) Date of filing: 25.09.86

(51) Int. Cl.[4]: A 61 K 7/08

(30) Priority: 27.09.85 GB 8523889

(43) Date of publication of application:
08.04.87 Bulletin 87/15

(84) Designated Contracting States:
AT BE CH DE FR GB IT LI NL

(71) Applicant: THE PROCTER & GAMBLE COMPANY
One Procter & Gamble Plaza
Cincinnati Ohio 45202 (US)

(84) Designated Contracting States:
BE CH DE FR IT LI NL AT

(71) Applicant: Procter & Gamble Limited
Hedley House
Gosforth Newcastle upon Tyne NE99 1EE (GB)

(84) Designated Contracting States: GB

(72) Inventor: Smith, Graeme Douglas Telfer
357 St. Leonard's Road
Windsor Berkshire (GB)

(74) Representative: Brooks, Maxim Courtney et al
Procter & Gamble (NTC) Limited Whitley Road
Longbenton
Newcastle-upon-Tyne NE12 9TS (GB)

(54) **Hair treatment.**

(57) A process of washing and conditioning hair comprising sequential application of a shampoo composition comprising surfactant and first antidandruff agent, and a rinse conditioning composition comprising cationic hair conditioning agent and second antidandruff agent. The first and second antidandruff agents together comprise a water-insoluble salt of l-hydroxy-2-pyridinethione (I) and a hydroxypyridone having the general formula II, or a salt thereof;

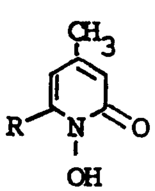

II

where R is an aliphatic or aromatic moiety having a $\pi$ factor of at least l.3. Preferred conditioning compositions also comprise a silicone hair conditioning agent.

EP 0 217 635 A2

**Description**

## HAIR TREATMENT

### Technical Field

The present invention relates to a process for washing and conditioning hair. In particular it relates to a washing and conditioning process providing improved formulation flexibility, aesthetic advantages as well as superior anti-dandruff effectiveness. In addition, it relates to hair conditioning compositions.

### Background

The use of antidandruff agents in shampoo and rinse conditioning compositions raises a number of problems which severely limit formulation flexibility. For example, although certain cationic surfactants are known to enhance the performance of antidandruff agents in rinse conditioning compositions, optimum conditioning performance is not easily achieved in the presence of the antidandruff agent. Where, on the other hand, conditioners are employed which perform satisfactorily in the presence of antidandruff agent, antidandruff efficacy is often adversely affected.

It would therefore, be valuable to provide a hair washing and conditioning process which provides for a greater degree of formulation flexibility without compromising either conditioning or antidandruff efficacy. In addition, it would be valuable to provide rinse conditioning compositions having improved antidandruff effectiveness and conditioning benefits.

The use of pyridinethione salts as antidandruff agents in shampoos and hair rinse is well known. US-A-3,236,733 discloses detergent compositions containing such salts. Other references include US-A-2,809,97l and US-A-3,723,325.

It is also known that certain l-hydroxy-2-pyridone derivatives are effective as antimicrobial agents with specific application to dandruff control. GB-A-l,440,975 is relevant in this respect.

Both the pyridinethione salts and the l-hydroxy-2 pyridones have been clinically proven to reduce the level of dandruff when used regularly over, for example, a four week period. Typically, there is a reduction in mean dandruff grades of about 50%-70% after four weeks twice weekly usage. Continued treatment can provide some further marginal improvements but on average, these materials reach a plateau in anti-dandruff effectiveness. Improvements in the level of dandruff reduction or in the rate at which dandruff reduction is achieved, would therefore be highly desirable.

The present invention is based in part on the finding that a wash process wherein certain pyridinethiones and hydroxypyridones are used sequentially in shampoo and rinse conditioning compositions provides superior formulation flexibility, improved conditioning effectiveness and most surprisingly, enhanced antidandruff efficacy.

In a further aspect of the invention, rinse conditioning compositions are provided based on a combination of specified cationic and hydrocarbon or silicone hair conditioning agents, together with certain hydroxypyridone antidandruff agents.

### Summary of the Invention

Accordingly, the present invention provides a process of washing and conditioning hair comprising the steps of:

(l) applying to the hair a shampoo composition comprising

(a) from 5% to 50% by weight thereof of surfactant selected from anionic, nonionic, amphoteric, and zwitterionic surfactants and mixtures thereof, and

(b) from 0.l% to 5% thereof of a first antidandruff agent selected from water-insoluble salts of l-hydroxy-2-pyridinethione (I), hydroxypyridones having a general formula II and salts thereof and mixtures of I and II:

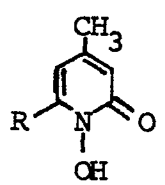

II

wherein R is an aliphatic or aromatic moiety having a $\pi$ factor of at least about l.3,

(2) rinsing the hair, and

(3) applying to the hair a rinse conditioning composition comprising

(a) from 0.05% to 5% by weight thereof of cationic hair conditioning agent and

(b) from 0.l% to 5% by weight thereof of a second antidandruff agent selected from water-insoluble salts of l-hydroxy-2-pyridonethione (I), hydroxypyridones having the general formula II and salts thereof, and mixtures of I and II;

and wherein the first and second antidandruff agents together comprise at least one pyridonethione of formula I and at least one hydroxypyridone of formula II or salt thereof.

The compositions used in the process of the invention together comprise two essential components, a pyridinethione component (I) and a hydroxypyridone component (II).

The pyridinethione component (I) can generally be defined as water-insoluble salts of l-hydroxy-2-pyridinethione which has the following structural formula in tautomeric form, the sulfur being attached to the No. 2 position in the pyridine ring.

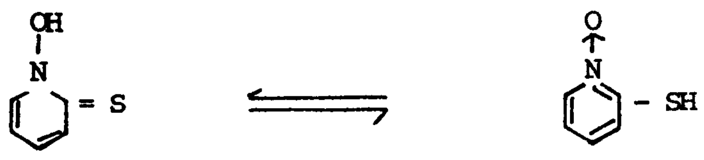

The salts result from substitution of the hydrogen of one of the tautomeric forms by the appropriate salt cation. Depending, of course, on the valence of the salt cation involved there may be more than one of the pyridinethione rings in the compound.

Preferred salts are formed from heavy metals such as zinc, tin, cadmium, magnesium, aluminium and zirconium. The preferred heavy metal is zinc. Other cations such as sodium are also suitable, however.

The pyridinethiones generally take the form of water-insoluble particles. These can have an individual particle size of at least l micrometer. Preferred materials, however, are pyridinethione crystals in the form of predominantly flat platelets which have a mean sphericity of less than about 0.65, preferably from about 0.20 to about 0.54 and a median particle size of at least about 2 micrometres, the particle size being expressed as the median equivalent diameter of a sphere of equal volume. The upper limit on mean particle size is not critical but generally can range up to about 25 micrometers, measured on the same basis. The median diameters are on a mass basis with 50% of the mass of particles falling on either side of the value given.

The diameter of a sphere of equivalent volume for a particle can be determined by a variety of sedimentation techniques which are based on Stokes' Law for the settling velocity of a particle in a fluid. Such techniques are described in Stockham, J.D. and Fochtman, E.G., Particle Size Analysis, Ann Arbor Science, l978. An approach for determining the median equivalent spherical diameter based on volume, $\bar{d}_v$, is shown in EP-A-34, 385, Example II.

The sphericity of a particle is also described by Stockham and Fochtman at page ll3 as $(d_v/d_s)^2$, where $d_v$ is the diameter of a sphere of equivalent volume, supra, and $d_s$ is the diameter of a sphere of equivalent area. As used herein, however, the mean sphericity is $(\bar{d}_v/\bar{d}_s)^2$, or the surface area of spheres having equivalent volume distribution divided by the actual surface area of particles as measured. A technique for determining actual surface area is shown in the examples using the BET technique described by Stockham and Fochtman at page l22.

From the viewpoint of anti-dandruff efficacy, the BET surface area herein preferably falls in the range from about 0.5 to about 3.5, more preferably from about l.0 to about $3.0 m^2/g$.

In preferred embodiments, pyridonethione anti-dandruff agents herein are at least partly in agglomerated form, the agglomerate size preferably being such that at least about 20% by weight, more preferably at least about 50% by weight of the pyridonethione is in the form of agglomerates having an equivalent spherical diameter of at least about 5 microns, preferably from about 7 to about 30 microns, more preferably from about l0 to about 20 microns. Agglomerate size is conveniently measured by sedimentation techniques using a Sedigraph 5000, Micrometrics Corp., the agglomerates being predispersed in water under low shear conditions. Agglomeration is preferred from the viewpoint of optimizing overall formulation efficacy.

The hydroxypyridone component (II) can generally be described as l-hydroxy-4-methyl-(lH)-pyridones having an aliphatic or aromatic moiety (R) at the 6 position thereof, wherein R has a $\pi$ factor of at least l.3, preferably from 2 to 6, more preferably from 3 to 5.5. The $\pi$ factor is a measure of the lipophilicity/hydrophilicity of the substituent and is defined in detail in the paper by W. Dittmar, E. Druckrey and H. Urbach, J. Med. Chem. l7(7), 753-6(l974) and references cited therein.

In structural terms, preferred R substituents are selected from linear and branched $C_3$-$C_{11}$, preferably $C_6$-$C_{11}$ alkyl and alkenyl groups, $C_5$-$C_8$ cycloalkyl groups, and aryl groups. In the above, cyclic moieties can also be substituted with one or more alkyl or alkenyl groups up to $C_4$. The R groups can further be substituted with halogen atoms. Of the above, preferred R moieties are cyclohexyl and 2,4,4-trimethyl pentyl, the latter being highly preferred.

The above mentioned compounds can be used both in the free form and as salts, for example, salts with organic bases or inorganic cations. Low molecular weight alkanolamines are especially preferred organic bases, for example triethanolamine.

The pyridinethione and hydroxypyridone antidandruff agents can be applied sequentially to the hair in either order. In a preferred embodiment, however, the first antidandruff agent is the pyridinethione (I) while the second antidandruff agent is the hydroxypyridone (II) or salt thereof. Preferably, the first and second antidandruff agents together comprise the pyridonethione and hydroxypyridone or salt thereof in a weight ratio of from about l0:l to about l:l0, preferably from about 5:l to about l:5. Individually, the pyridinethiones and

hydroxypyridones each generally comprise from about 0.l% to about 5%, preferably from about 0.l% to about 3%, more preferably from about 0.2% to about 2% by weight of the corresponding composition.

One essential component of the antidandruff shampoo composition is a surfactant selected from anionic, nonionic, amphoteric and zwitterionic types. The term "surfactant" as used herein is intended to denote soap and nonsoap surfactants. The surfactant component usually comprises from about 5% to about 50% by weight of the composition, preferably from about l0% to about 20%.

Examples of suitable soaps are the sodium, potassium, ammonium and alkanol ammonium salts of higher fatty acids (those having l0-20 carbon atoms). Anionic nonsoap surfactants can be exemplified by the alkali metal salts of organic sulfuric reaction products having in their molecular structure an alkyl radical containing from 8 - 22 carbon atoms and a sulfonic acid or sulfuric acid ester radical (included in the term is the alkyl portion of higher acyl radicals). Preferred are the sodium, ammonium, potassium or triethanolamine alkyl sulfates, especially those obtained by sulfating the higher alcohols ($C_8$ - $C_{18}$) carbon atoms), sodium coconut oil fatty acid monoglyceride sulfates and sulfonates; sodium or potassium salts of sulfuric acid esters of the reaction product of l mole of a higher fatty alcohol (e.g., tallow or coconut oil alcohols) and l to l2 moles of ethylene oxide; sodium or potassium salts of alkyl phenol ethylene oxide either sulfate with l to l0 units of ethylene oxide per module and in which the alkyl radicals contain from 8 to l2 carbon atoms, sodium alkyl glyceryl ether sulfonates; the reaction product of fatty acids having from l0 to 22 carbon atoms esterified with isethionic acid and neutralized with sodium hydroxide; water soluble salts of condensation products of fatty acids with sarcosine; and others known in the art.

Nonionic surfactants can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

l. The polyethlene oxide condensates of alkyl phenols, e.g., the condensation products of alkyl phenols having an alkyl group containing from about 6 to l2 carbon atoms in either a straight chain or branched chain configuration, with ethylene oxide, the said ethylene oxide being present in amounts equal to l0 to 60 moles of ethylene oxide per mole of alkyl phenol. The alkyl substituent in such compounds may be derived from polymerixed propylene, diisobutylene, octane, or nonane, for example.

2. Those derived from the condensation of ethylene oxide with the product resulting from the reaction of propylene oxide and ethylene diamine products which may be varied in composition depending upon the balance the hydrophobic and hydrophilic elements which is desired. For example, compounds containing from about 40% to about 80% polyoxyethylene by weight and having a molecular weight of from about 5,000 to about ll,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to l8 carbon atoms, in either straight chain or branched chain configuration, with ethylene oxide, e.g., a coconutalcohol ethylene oxide condensation having from l0 to 30 moles of ethylene oxide per mole of coconut alcohol, the coconut alcohol fraction having from l0 to l4 carbon atoms.

4. Lomg chain tertiary amine oxides corresponding to the following general formula:

$R_1R_2R_3N \rightarrow 0$

wherein $R_1$ contains an alkyl, alkenyl or monohydroxy alkyl radical of from about 8 to about l8 carbon atoms, from 0 to about l0 ethylene oxide moieties, and from 0 to l glyceryl moiety, and $R_2$ and $R_3$ contain from l to about 3 carbon atoms and from 0 to about l hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyldi(2-hydroxyethyl)amine oxide, dimethyloctylamine oxide, dimethyldecylamine oxide, dimethyltetradecylamine oxide, 3,6,9-trioxaheptadecyldiethylamine oxide,
di(2-hydroxyethyl)-tetradecylamine oxide,
2-dodecoxyethyldimethylamine oxide,
3-dodeoxycy-2-hydroxypropyldi(3-hydroxypropyl)amine oxide,
dimethylhexadecylamine oxide.

Zwitterionic surfactants can be exemplified by those which can be broadly described as derivatives of aliphatic quaternary ammonium, phosphonium, and sulfonium compounds, in which the aliphatic radicals can be straight chain or branched, and wherein one of the aliphatic substituents contains from about 8 to l8 carbon atoms and one contains an anionic water-solubilizing group, e.g., carboxy, sulfonate, sulfate, phosphate, or phosphonate. A general formula for these compounds is:

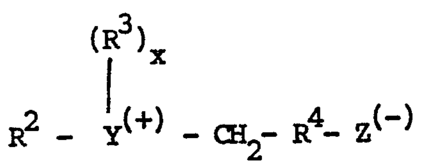

wherein $R^2$ contains an alkyl, alkenyl, or hydroxy alkyl radical of from about 8 to about l8 carbon atoms, from 0 to about l0 ethylene oxide moieties and from 0 to l glyceryl moiety; Y is selcted from nitrogen, phosphorus, and

sulfur atoms, $R^3$ is an alkyl or monohydroxyalkyl group containing I to about 3 carbon atoms; x is I when Y is a sulfur atom and 2 when Y is a nitrogen or phosphorus atom; $R^4$ is an alkylene or hydroxyalkylene of from I to about 4 carbon atoms and Z is a radical selected from carboxylate, sulfonate, sulfate, phosphonate. and phosphate groups.

Examples of amphoteric surfactants which can be used in the compositions of the present invention can be broadly described as derivatives of aliphatic secondary and tertiary amines in which the aliphatic radical can be straight chain or branched and wherein one of the aliphatic substituents contains from about 8 to about I8 carbon atoms and one contains an anionic water solubilizing group, e.g., carboxy, sulfonate, sulfate, phosphate, or phosphonate. Examples of compounds falling within this definition are sodium 3-dodecylaminopropionate, sodium 3-dodecylaminopropane sulfonate, N-alkyltaurines such as the one prepared by reacting dodecylamine with sodium isethionate according to the teaching of US-A-2,658,072, N-higher alkyl aspartic acids such as those produced according to the teaching of US-A-2,438,09I, and the products sold under the trade name "Miranol" and described in US-A-2,528,378.

Many additional nonsoap surfactants are described in McCUTCHEON'S DETERGENTS AND EMULSIFIERS, I979 ANNUAL, published by Allured Publishing Corporation.

The above-mentioned surfactants can be used alone or in combination.

The shampoo compositions can optionally be complemented by low levels (up to about 4%) of cationic surfactants.

Many cationic surfactants are known to the art. By way of example, the following may be mentioned:
dodecyltrimethylammonium chloride;
nonylbenzylethyldimethylammonium nitrate;
tetradecylpyridinium bromide;
laurylpyridinium chloride;
cetylpyridinium chloride;
laurylpyridinium chloride;
laurylisoquinolium bromide;
ditallow(hydrogenated)dimethyl ammonium chloride;
dilauryldimethylammonium chloride; and
stearalkonium chloride.

The shampoo composition will generally contain a suspending agent. The suspending agent is preferably substantially crystalline in form. Also, preferred suspending agents are non-polymeric and non-clay in nature, such materials being found to have a deleterious effect on overall anti-dandruff efficacy. Accordingly, the shampoo compositions are preferably substantially free of nonionic and anionic polymeric and clay-type suspending agents. By "substantially free" it is meant not more than about 20 ppm of these materials. Preferred suspending agents include ethylene glycol and glycerol esters of fatty acids having from about I6 to about 22 carbon atoms. Both ethylene glycol mono and distearate are examples of these esters.

Other useful suspending agents are alkanolamides of fatty acids having from about I6 to about 22 carbon atoms, preferably about I6 to I8 carbon atoms. Suitable examples of these agents include stearic monoethanolamide, stearic diethanolamide, stearic monoisopropanolamide, stearic diethanolamide distearate and mixtures thereof.

Still other suitable suspending agents are $C_{16}$-$C_{22}$ alkyl dimethylamine oxides such as stearyl dimethyl amine oxide and $C_{16}$-$C_{22}$ fatty acid amidoalkylamines such as stearamidoethyldiethylamine. Mixtures of suspending agents are also acceptable, for example, a mixture of glyceryl stearate and stearamidoethyldiethylamine. When present, these suspending agents are at levels of from about I% to about 7%, preferably from about 2% to about 6%. These agents mat also serve to give pearlescence to the product.

The shampoo composition can obtain a variety of non-essential optional ingredients suitable for rendering such compositions more stable and desirable. Such conventional optional ingredients are well known to those skilled in the art, e.g., pearlescent materials such as bismuth oxychloride, guanine and titanium dioxide coated mica, preservatives such as benzyl as benzyl alcohol, methyl paraben, propyl paraben and imidazolidinyl urea; and sequestering agents such as disodium ethylenediamine tetraacetate.

An amide is a preferred optional component present in the shampoo composition. Any of the alkanolamides of fatty acids useful in shampoo compositions can be used. Generally, these include mono and diethanolamides of fatty acids having from about 8 to about I4 carbon atoms. Preferred compounds include coconut monoethanolamide, lauric or coconut diethanolamide, and mixtures thereof. The amide is present at a level of from about I.0% to about 4.0%. It is believed that a more richer lathering, more stable product results when amides of these types are combined with the other components of the invention at hand.

Another preferred but optional component herein is an electrolyte. The electrolyte should have an equivalent weight of less than about I50, preferably less than about I00 and is preferably selected from alkali metal, alkaline earth metal, ammonium and substituted ammonium chlorides, sulphates and citrates. The level of electrolyte is preferably from about 20 to about I50 milliequivalents %.

Minor ingredients such as perfumes, dyes and coloring agents can also be added to the instant compositions to improve their consumer acceptability. If present, such agents generally comprise from about 0.I% to 2.0% by weight of the composition.

The shampoos herein are preferably in the form of liquids or creams in which is the principle diluent. The level of water in the compositions is typically from about 35% to about 90% by weight.

The rinse conditioning composition comprises a mixture of second antidandruff agent together with a cationic hair conditioning agent.

The cationic hair conditioning agent may be either a quaternary ammonium salt ot the salt of a fatty amine.

Quaternary ammonium salts have the formulae $R_1R_2R_3R_4N+\ X-$, wherein $R_1$ is hydrogen, an aliphatic group of from l to 22 carbon atoms, or aromatic, aryl or alkaryl groups having from l2 to 22 carbon atoms; $R_2$ is an aliphatic group having l-22 carbon atoms; $R_3$ and $R_4$ are each alkyl groups of from l to 3 carbon atoms, and X is an anion selected from halogen, acetate, phosphate, nitrate and methyl sulfate radicals. The aliphatic groups may contain, in addition to carbon and hydrogen atoms, ether linkages as well as amido groups.

Preferred quaternary ammonium salts include the dialkyl dimethyl ammonium chlorides, wherein the alkyl groups have from l2 to 22 carbon atoms and are derived from long-chain fatty acids, such as hydrogenated tallow. The term 'tallow' refers to fatty alkyl gorups derived from tallow fatty acids. Such fatty acids give rise to quaternary compounds wherein $R_1$ and $R_2$ have predominately from l6 to l8 carbon atoms. The term 'coconut' refers to fatty acid groups from coconut oil fatty acids. The coconut-alkyl $R_1$ and $R_2$ groups have from 8 to l8 carbon atoms and predominate in $C_{12}$ to $C_{14}$ alkyl groups.

Representative examples of quaternary ammonium salts include ditallow dimethyl ammonium chloride; ditallow dimethyl ammonium methyl sulfate; dihexadecyl dimethyl ammonium chloride; di(hydrogenated tallow) dimethyl ammonium chloride; dioctadecyl dimethyl ammonium chloride; dieicosyl dimethyl ammonium chloride; didocosyl dimethyl ammonium chloride; di(hydrogenated tallow) dimethyl ammonium acetate; ditallow dipropyl ammonium phosphate; ditallow dimethyl ammonium nitrate; di(coconutalkyl) dimethyl ammonium chloride; and stearyl dimethyl benzyl ammonium chloride. Ditallow dimethyl ammonium chloride, dicetyl dimethyl ammonium chloride, stearyl dimethyl benzyl ammonium chloride and cetyl trimethyl ammonium chloride are preferred quaternary ammonium salts useful herein. Di-(hydrogenated tallow) dimethyl ammonium chloride is a particularly preferred quaternary ammonium salt.

Also suitable herein are the di-$C_{12}$-$C_{22}$ alkyl or alkenyl quaternary imidazolinium salts, eg l-methyl-l-[(stearylamido)ethyl)]-2-heptadecyl-4, 5-dihydroimidazolinium chloride; 1-methyl-l-[(parmitylamido)-ethyl]-2-octadecyl-4, 5-dihydroimidazolinium chloride and l-methyl-l-[(tallowamido)-ethyl]-2-tallow-imidazolium methyl sulfate.

Included as suitable cationic hair conditioner herein are salts of fatty amines. As used herein the amines may be primary, secondary or tertiary but the alkyl preferably have from l2-22 carbon atoms. Preferred are the primary and secondary amines with the primary being the most preferred. Diamines having a long chain alkyl group may also be used. Examples of amines suitable for use include dimethyl stearamine, dimethyl soyamine, stearylamine, soyamine, myristylamine, tridecylamine, ethyl stearylamine, N-tallow propanediamine, ethoxylated (5 moles E.O.) stearylamine, dihydroxyethyl stearylamine and arachidylbehenylamine. Suitable amine salts include the halogen, acetate, phosphate, nitrate, citrate, lactate and alkyl sulfate salts. Such salts include stearylamine hydrochloride, soyamine chloride, stearylamine formate and N-tallowpropane diamine di-chloride and strearamidopropyl dimethylamine citrate.

The cationic hair conditioning agent is present at a level of from 0.05% to 5% by weight, more preferably from 0.l% to 2% by weight.

The conditioning composition preferably also comprises a volatile hydrocarbon or silicone fluid conditioning agent. The silicones are preferably polydimethylsiloxanes.

The volatile hydrocarbon and polydimethylsiloxane agents useful in the present compositions having a boiling point in the range of 99°C to 260°C and have a solubility in water of less than 0.l%. The hydrocarbons are either straight or branched chain and contain from l0 to l6, preferably from l2 to l6 carbon atoms. Examples of suitable hydrocarbons are decane, dodecane, tetradecane, tridecane and mixtures thereof.

The polydimethylsiloxanes are either cyclic or linear polydimethylsiloxanes. The number of silicon atoms in the cyclic silicones is from 3 to 7, preferably 4 or 5. The linear polydimethylsiloxanes have from 3 to 9 silicon atoms.

Silicones of the above type, both cyclic and linear, are offered by Dow Corning Corpoartion, Dow Corning 344, 345 and 200 fluids, Union Carbide, Silicone 7202 and Silicone 7l58, and Stauffer Chemical, SWS-033l4.

The linear polydimethylsiloxanes generally have viscosities of less than 5 centistokes at 25°C while the cyclic materials have viscosities less than l0 centistokes. A description of volatile silicones is found in Todd and Byers, 'Volatile Silicone Fluids for Cosmetics', Cosmetics and Toiletries, Vol. 9l, January l976, pp. 29-32.

The liquid hair conditioning agent is preferably present at a level of from 0.l% to l5% by weight, preferably from l% to l0% by weight, and especially from l% to 6% by weight. The polydimethylsiloxanes are the preferred agents.

The conditioning composition preferably also comprises a non-volatile silicone fluid or gum having a viscosity of from l0 to 2,500,000, preferably l0 to l00,000 $mm^2s^{-1}$(cST) at 25°C. The non-volatile silicone can be a polyalkylsiloxane, a polyalkylarylsiloxane or a polyether siloxane copolymer. Preferred are polydimethylsiloxanes, for example, Dow Corning 200.

The conditioning composition herein preferably also contain a water-soluble, substantially nonionic thickening agent. The polymers are those which exhibit viscosities exceeding 200 Pa.s (200 poises) at $l0^{-2}sec^{-1}$, when their viscosities at 500 $sec^{-1}$ are less than 0.9 Pa.s (9 poises). Preferred are those polymers which show about a l00 fold increase in viscosity over the shear rate range of 500 to $l0^{-2}sec^{-1}$. The polymers are selected from polyoxyethylene, guar gum, methylcellulose, methyl hydroxypropyl cellulose, hydroxyethyl cellulose, locust bean gum, hydroxypropyl guar gum, hydroxypropyl cellulose, starch, hydroxyethyl amylose and starch amylose and mixtures thereof. Preferred polymers are guar gum and hydroxypropyl guar gum.

The water soluble thickening agent is preferably present in the present compositions at a level of from 0.1% to 8% by weight, more preferably from 0.5% to 6.0% by weight.

In addition to the above described essential components the conditioning composition may contain a wide variety of optional components, for example, hydrophobic polymers such as polyvinyl isobutyl ether, waxes such as cetyl alcohol and paraffin, oils such as mineral oil and isopropyl myristate, low levels (0.05% to 5%) of nonionic, anionic and amphoteric surfactants such as ceteareth-20, steareth-20, ceteth-2, sorbitan monoesters sodium tallow alkyl sulfate and tallow betaine, and from 0.01% to 10% of polyalkylene oxide modified dimethylpolysiloxanes as disclosed in EP-A-85301672-3, for example Dow Corning 190 silicone surfactant, Rhodorsil 70646 Fluid, Silvet L-720 and Silvet L-7002.

The shampoo and rinse conditioning compositions both have a pH generally in the range from about 3 to about 8. Preferred compositions are acidic however, highly preferred compositions having a pH in the range from about 3.5 to about 6.5, especially from about 4 to about 6. The pH is measured on a 1% solution basis. A preferred pH regulating agent is a citric acid/sodium citrate buffer. Other acidulating agents such as phosphoric acid, succinic acid etc. can also be used, however.

In use, the shampoo composition is normally applied to prewetted hair in an amount of from about 1 to about 60, preferably from about 5 to about 30g per application, the hair is rinsed, the rinse conditioner is then applied in an amount of from 1 to about 60, preferably from about 5 to about 30g per application, and finally the hair is rinsed again.

The shampoo and rinse conditioner can be provided herein in the form of a kit comprising the two component compositions packaged either in separate containers or in two or more compartments of a multicompartment container. Instructions describing the sequential application of the two compositions are provided as part of the kit.

The following Examples further describe and demonstrate the preferred embodiments within the scope of the present invention. Unless otherwise indicated, all percentages herein are by weight of the shampoo or conditioner composition respectively.

In the Examples which follow, the abbreviations have the following designations.

ZPT1 Zinc Pyridinethione
Median Equivalent Spherical Diameter = 5.4 microns
Means Sphericity = 0.25

ZPT2 Zinc Pyridinethione
Median Equivalent Spherical Diameter = 1.25 microns
Mean Sphericity = 0.75

ZPT3 Agglomerated Zinc Pyridinethione - 20% having an equivalent spherical diameter of at least 5 microns

ZPT4 Agglomerated Zinc Pyridinethione platelets - 50% having an equivalent spherical diameter of at least 13 microns

TMPP 1-hydroxy-4-methyl-6-(2,4,4-trimethyl pentyl)-2-(1H)-pyridone, triethanolamine salt
CHP 1-hydroxy-4-methyl-6-cyclohexyl-2-(1H)-pyridone, triethanolamine salt.
$NH_4AS$ Ammonium $C_{12}$ alkyl sulfate (29% aq. soln.)
TEA AS Triethanolamine $C_{12}$ alkyl sulfate (35% aq. soln,)
$NH_4$ $AE_2S$ Ammonium $C_{12}$ alkyl $(EO)_2$ sulfate (24% active)
AGS Sodium alkyl glyceryl ether sulfonate (60%)
MEA Coconut Monoethanoamide
DEA Lauric Diethanolamide
LS Sodium N-lauaroyl sarcosinate
CSA N-Cocoyl Sarcosine Acid
EGDS Ethylene glycol distearate
DTDMAC Ditallow dimethyl ammonium chloride
DCDMAC Dicetyl dimethyl ammonium chloride
DTMA Ditallowmethylamine hydrochloride
STEDBAC Stearyl dimethyl benzyl ammonium chloride
CETAC Cetyl trimethyl ammonium chloride
DC 345 Cyclic polydimethylsiloxane having 5 dimethylsiloxane groups - from Dow Corning
DC 190 Dow Corning 190 silicone surfactant
Jaguar HP-60 Hydroxypropyl guar gum
Lexamine S-13 Stearamidopropyldimethylamime, sold by Inolex Corporation
Polyox WSR-205 Polyoxyethylene - supplied by Union Carbide
Silicone Polydimethylsiloxane oil (12,500 cs)
Kathon CG Presevative offered by Rohm and Huas

<u>Examples I to VII</u>

The following shampoo and conditioning compositions are prepared:

| Shampoo Composition | I | II | III | IV | V | VI | VII |
|---|---|---|---|---|---|---|---|
| ZPT1 | - | - | - | - | 1 | - | - |
| ZPT2 | - | 1 | - | - | - | - | - |
| ZPT3 | - | - | 1 | - | - | - | - |
| ZPT4 | 1 | - | - | 1 | - | - | - |
| TMPP | - | - | - | - | - | 1 | - |
| CHP | - | - | - | - | - | - | 1 |
| $NH_4AS$ | 55 | - | - | - | 28 | 45 | 25 |
| TEA AS | - | 50 | - | 55 | - | - | - |
| $NH_4$ $AE_2S$ | - | 3 | - | - | 40 | 2 | 35 |
| AGS | - | - | 60 | - | - | - | - |
| MEA | 3 | 2 | - | 3 | 4 | 3 | 2 |
| DEA | - | - | 2 | - | - | - | - |
| LS | - | - | 12 | - | - | - | - |
| CSA | - | - | 1 | - | - | - | - |
| EGDS | 5 | 4 | 3 | 5 | 3 | 5 | 5 |
| Sodium Chloride | - | - | 5 | - | - | - | - |
| Sodium Citrate | 0.5 | 0.5 | 0.6 | 0.8 | 0.7 | 0.8 | 0.5 |
| Citric Acid | 0.2 | 0.2 | 0.3 | 0.6 | 0.4 | 0.5 | 0.2 |
| Colour solution | 0.1 | 0.1 | 0.12 | 0.2 | 0.1 | 0.1 | 0.1 |
| Perfume | 0.5 | 0.3 | 0.5 | 0.6 | 0.5 | 0.5 | 0.5 |
| Water | To 100% | | | | | | |

The shampoo compositions are prepared as follows. All materials except the preservative, perfume and antidandruff agent are added to the water maintained at a temperature of from 65° C to 74° C. The mix is then cooled to 38° C in a heat-exchanger at which point the antidandruff agent is added. The mix is then further cooled to 27° C at which point the preservative and fragrance are added.

| Conditioning Composition | I | II | III | IV | V | VI | VII |
|---|---|---|---|---|---|---|---|
| ZPT1 | - | - | - | - | - | - | 0.3 |
| ZPT4 | - | - | - | - | - | 0.3 | - |
| TMPP | 0.3 | - | 0.3 | 0.3 | 0.3 | - | - |
| CHP | - | 0.3 | - | - | - | - | - |
| DC345 | 2 | 8 | - | 6 | - | 2 | - |
| DC190 | 0.5 | - | - | 2 | - | 0.5 | - |
| Dodecane | - | - | 10 | - | - | - | - |
| Jaguar HP-60 | - | 1.1 | 1.75 | - | - | - | - |
| DTDMAC | 0.75 | 0.24 | - | - | - | 0.75 | - |
| STEDBAC | - | - | - | - | 1 | - | 1 |
| DCDMAC | - | - | - | 1 | - | - | - |
| CETAC | - | - | - | - | 1 | - | 1 |
| DTMA | - | - | 0.25 | - | - | - | - |
| Glycerol-monostearate | - | - | - | 3 | - | - | - |
| Ethanol | - | 8 | - | - | - | - | - |
| Silicone | - | - | - | 1 | - | - | - |
| Hydroxyethyl Cellulose | 0.5 | - | - | - | - | 0.5 | - |
| Polyox WSR-205 | - | - | 0.1 | - | - | - | - |
| Cetyl alcohol | 1.25 | - | - | - | 1.3 | 1.25 | 1.3 |
| Stearyl alcohol | 1.25 | - | - | - | 1.3 | 1.25 | 1.3 |
| Lexamine S-13 | 0.75 | - | - | - | - | 0.75 | - |
| Citric acid | 0.13 | - | - | - | 0.1 | 0.13 | 0.1 |
| Sodium chloride | - | - | - | - | 0.1 | - | 0.1 |
| Kathon CG | 0.03 | 0.03 | 0.03 | 0.03 | 0.03 | 0.03 | 0.03 |
| Perfume | 0.5 | 0.5 | 0.5 | 0.5 | 0.5 | 0.5 | 0.5 |
| Water | To 100% | | | | | | |

The conditioning compositions are made as follows. All materials, except the preservative, fragrance, and antidandruff agent are added to the distilled water, maintained at a temperature of from 65°C to 74°C. This mixture is then stirred for 15 minutes. After the solution is cooled to approximately 49°C, the antidandruff agent, fragrance, perservative are added. The mixtures are then cooled to approximately 38°C and milled under high shear for approximately 2 minutes using a conventional milling apparatus.

In use, approximately 20g of shampoo is applied to prewetted hair which is washed for about 30 seconds. The hair is rinsed and approximately 20g of conditioning composition is then applied to the hair. The conditioning composition is allowed to stand for 1 minute and is then rinsed from the hair.

The washing and conditioning processes of Examples I to VII all provided excellent antidandruff

performance and simultaneous conditioning performance.

## Claims

1. A process of washing and conditioning hair comprising the steps of:
(1) applying to the hair a shampoo composition comprising
(a) from 5% to 50% by weight thereof of surfactant selected from anionic, nonionic, amphoteric, and zwitterionic surfactants and mixtures thereof, and
(b) from 0.1% to 5% thereof of a first antidandruff agent selected from water-insoluble salts of 1-hydroxy-2-pyridinethione (I), hydroxypyridones having the general formula II and salts thereof and mixtures of I and II:

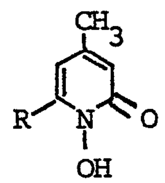

II

wherein R is an aliphatic or aromatic moiety having a $\pi$ factor of at least about 1.3,
(2) rinsing the hair; and
(3) applying to the hair a rinse conditioning composition comprising
(a) from 0.05% to 5% by weight thereof of cationic hair conditioning agent and
(b) from 0.1% to 5% by weight thereof of a second antidandruff agent selected from water-insoluble salts of 1-hydroxy-2-pyridonethione (I), hydroxypyridones having the general formula II and salts thereof, and mixtures of I and II;
and wherein the first and second antidandruff agents together comprise at least one pyridonethione of formula I and at least one hydroxypyridone of formula II or salt thereof.

2. A process according to Claim 1 wherein the pyridinethione (I) is selected from zinc, tin, magnesium, aluminium, cadmium, zirconium and sodium salts of 1-hydroxy-2-pyridinethione.

3. A process according to Claim 1 or 2 wherein the pyridinethione (I) is the zinc salt of 1-hydroxy-2-pyridinethione.

4. A process according to any of Claims 1 to 3 wherein the pyridinethione (I) has a particle size, defined as the mass median equivalent spherical diameter, of at least 1 micrometre.

5. A process according to Claim 4 wherein the pyridinethione (I) has a particle size in the range from 2 to 25 micrometres.

6. A process according to any of Claims 1 to 5 wherein the pyridinethione (I) is in the form of predominantly flat platelets having a mean sphericity of less than 0.65.

7. A process according to any of Claims 1 to 6 wherein the pyridinethione (I) is at least partly in agglomerated form.

8. A process according to Claim 7 wherein at least 20% of the pyridinethione (I) is in the form of agglomerates having an equivalent spherical diameter of at least 5 microns.

9. A process according to any of Claims 1 to 8 wherein R is selected from linear and branched, $C_3$-$C_{11}$ alkyl and alkenyl groups, $C_5$-$C_8$ cycloalkyl groups, and aryl groups.

10. A process according to Claim 9 wherein R is 2,4,4-trimethylpentyl.

11. A process according to Claim 10 wherein the hydroxypyridone (II) is in the form of an alkanolamine salt.

12. A process according to any of Claims 1 to 11 wherein the first and second antidandruff agents together comprise the pyridinethione (I) and hydroxypyridone (II) in weight ratio of from 10:1 to 1:10.

13. A process according to any of Claims 1 to 12 wherein the shampoo composition has a pH (1% aqueous solution) in the range from 4 to 6.

14. A process according to any of Claims 1 to 13 wherein the first antidandruff agent is 1-hydroxy-2-pyridinethione (I) and the second antidandruff agent is hydroxypyridone (II) or salt thereof.

15. A process according to any of Claims 1 to 14 wherein the shampoo composition comprises an anionic surfactant selected from alkyl sulfates, ethoxylated alkyl sulfates, alkyl glyceryl ether sulfonates and mixtures thereof.

16. A process according to any of Claims 1 to 15 wherein the shampoo composition additionally incorporates a non-polymeric, non-clay suspending agent.

17. A process according to Claim 16 wherein the suspending agent is selected from $C_{16}$-$C_{22}$ fatty acid esters of glycerol or ethylene glycol, $C_{16}$-$C_{22}$ fatty acid alkanolamides, $C_{16}$-$C_{22}$ fatty acid amido alkylamines and $C_{16}$-$C_{22}$ alkyl amineoxides.

18. A process according to any of Claims 1 to 17 wherein the shampoo composition additionally comprises from 20 to 150 milliequivalents % of electrolyte.

19. A process according to any of Claims 1 to 18 wherein the rinse conditioning composition additionally comprises from 0.1% to 15% of hydrocarbon or silicone hair conditioning agent.

20. A process according to 19 wherein the silicone hair conditioning agent is selected from cyclic polydimethylsiloxanes having from 3 to 7 silicon atoms and linear polydimethylsiloxanes having from 3 to 9 silicon atoms.

21. A hair shampoo and conditioning multicomponent kit comprising

(A) a first component in the form of a hair shampoo composition comprising:

(a) from 6% to 50% by weight thereof of surfactant selected from anionic, nonionic, amphoteric and zwitterionic surfactants and mixtures thereof, and

(b) from 0.1% to 5% thereof of a first antidandruff agent selected from water-insoluble salts of 1-hydroxy-2-pyridinethione (I), hydroxypyridones having the general formula II and salts thereof, and mixtures of I and II:

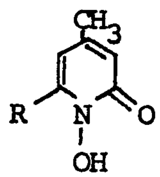

II

wherein R is an aliphatic or aromatic moiety having a $\pi$ factor of at least about 1.3,

(B) a second component in the form of a rinse conditioning composition comprising

(a) from 0.1% to 5% by weight thereof of cationic hair conditioning agent and

(b) from 0.1% to 5% by weight thereof of a second antidandruff agent selected from water-insoluble salts of 1-hydroxy-2-pyridonethione (I), hydroxypyridones having the general formula II and salts thereof, and mixtures of I and II;

and wherein the first and second antidandruff agents together comprise at least one pyridonethione of formula I and at least one hydroxypyridone of formula II or salt thereof.

22. A hair rinse conditioning composition comprising:

(a) from 0.1% to 5% by weight of cationic hair conditioning agent

(b) from 0.1% to 5% by weight of antidandruff agent comprising a hydroxypyridone having the general formula II or salt thereof and

(c) from 0.1% to 15% of hydrocarbon or silicone hair conditioning agent.